# EUROPEAN PATENT APPLICATION

(11) **EP 1 767 945 A1**
(43) Date of publication of application: **28.03.2007**
(21) Application number: 05025639.5
(22) Date of filing: 24.11.2005
(51) Int. Cl.: G01N 33/574, C07K 14/72

(54) **Phosphorylated or non-phosphorylated mPR as diagnostic marker or therapeutic target**

(30) Priority: 26.09.2005 EP 05020917
(71) Applicant: ProteoSys AG, 55129 Mainz (DE)
(72) Inventor: Cahill, Michael, 55296 Loerzweiler (DE); Schrattenholz, André, 55129 Mainz (DE); Kurek, Raffael, 64807 Dieburg (DE); Wallwiener, Diethelm, 72076 Tübingen (DE); Neubauer, Hans, 72411 Bodelshausen (DE); Clare, Susan, Chicago, IL 60611 (US)
(74) Representative: Patentanwälte Ruff, Wilhelm, Beier, Dauster & Partner

(57) **Abstract**

The invention relates to the use of phosphorylated and/or non-phosphorylated membrane associated progesterone receptor component 1 (mPR), in particular of at least one isoform thereof, and to other proteins, in particular isoforms thereof, as diagnostic markers and/or therapeutic targets for diseases associated with aberrant biological phenotypes, reagents for influencing mPR and/or the abundance and/or activity of said other proteins and to the use of an appropriate assay.

SEQUENCE LISTING

<110>  ProteoSys AG

<120>  Detection of phosphorylated proteins II

<130>  P45871 EP

<160>  10

<170>  PatentIn version 3.3

<210>  1
<211>  15
<212>  PRT
<213>  Homo sapiens


<220>
<221>  Seq 1
<222>  (1)..(15)

<223>  Sequence Description: SEQ ID NO:1:
<400>  1

Gln Pro Ala Ala Ser Gly Asp Ser Asp Asp Asp Glu Pro Pro Pro
1               5                   10                  15


<210>  2
<211>  15
<212>  PRT
<213>  Homo sapiens


<220>
<221>  Seq 2
<222>  (1)..(15)

<223>  Sequence Description: SEQ ID NO:2:
<400>  2

Asp Ser Asp Asp Asp Glu Pro Pro Pro Leu Pro Arg Leu Lys Arg
1               5                   10                  15


<210>  3
<211>  15
<212>  PRT
<213>  Homo sapiens


<220>
<221>  Seq 3
<222>  (1)..(15)

<223>  Sequence Description: SEQ ID NO:3:
<400>  3

Lys Gly Arg Lys Phe Tyr Gly Pro Glu Gly Pro Tyr Gly Val Phe
1               5                   10                  15

<210> 4
<211> 15
<212> PRT
<213> Homo sapiens

<220>
<221> Seq 4
<222> (1)..(15)

<223> Sequence Description: SEQ ID NO:4:
<400> 4

Phe Tyr Gly Pro Glu Gly Pro Tyr Gly Val Phe Ala Gly Arg Asp
1               5                   10                  15

<210> 5
<211> 15
<212> PRT
<213> Homo sapiens

<220>
<221> Seq 5
<222> (1)..(15)

<223> Sequence Description: SEQ ID NO:5:
<400> 5

Lys Glu Ala Leu Lys Asp Glu Tyr Asp Asp Leu Ser Asp Leu Thr
1               5                   10                  15

<210> 6
<211> 15
<212> PRT
<213> Homo sapiens

<220>
<221> Seq 6
<222> (1)..(15)

<223> Sequence Description: SEQ ID NO:6:
<400> 6

Ser Asp Trp Glu Ser Gln Phe Thr Phe Lys Tyr His His Val Gly
1               5                   10                  15

<210> 7
<211> 15
<212> PRT
<213> Homo sapiens

<220>
<221> Seq 7
<222> (1)..(15)

<223> Sequence Description: SEQ ID NO:7:
<400> 7

Glu Gly Glu Glu Pro Thr Val Tyr Ser Asp Glu Glu Glu Pro Lys
1               5                   10                  15

<210>  8
<211>  15
<212>  PRT
<213>  Homo sapiens

<220>
<221>  Seq 8
<222>  (1)..(15)

<223>  Sequence Description: SEQ ID NO:8:
<400>  8

Gly Glu Glu Pro Thr Val Tyr Ser Asp Glu Glu Glu Pro Lys Asp
1               5                   10                  15

<210>  9
<211>  194
<212>  PRT
<213>  Homo sapiens

<220>
<221>  Seq 9
<222>  (1)..(194)

<223>  Sequence Description: SEQ ID NO:9:
<400>  9

Ala Ala Glu Asp Val Val Ala Thr Gly Ala Asp Pro Ser Asp Leu Glu
1               5                   10                  15

Ser Gly Gly Leu Leu His Glu Ile Phe Thr Ser Pro Leu Asn Leu Leu
            20                  25                  30

Leu Leu Gly Leu Cys Ile Phe Leu Leu Tyr Lys Ile Val Arg Gly Asp
        35                  40                  45

Gln Pro Ala Ala Ser Gly Asp Ser Asp Asp Asp Glu Pro Pro Pro Leu
    50                  55                  60

Pro Arg Leu Lys Arg Arg Asp Phe Thr Pro Ala Glu Leu Arg Arg Phe
65                  70                  75                  80

Asp Gly Val Gln Asp Pro Arg Ile Leu Met Ala Ile Asn Gly Lys Val
                85                  90                  95

Phe Asp Val Thr Lys Gly Arg Lys Phe Tyr Gly Pro Glu Gly Pro Tyr
            100                 105                 110

Gly Val Phe Ala Gly Arg Asp Ala Ser Arg Gly Leu Ala Thr Phe Cys
        115                 120                 125

Leu Asp Lys Glu Ala Leu Lys Asp Glu Tyr Asp Asp Leu Ser Asp Leu
    130                 135                 140

Thr Ala Ala Gln Gln Glu Thr Leu Ser Asp Trp Glu Ser Gln Phe Thr
145                 150                 155                 160

Phe Lys Tyr His His Val Gly Lys Leu Leu Lys Glu Gly Glu Glu Pro
                165                 170                 175

Thr Val Tyr Ser Asp Glu Glu Glu Pro Lys Asp Glu Ser Ala Arg Lys
            180                 185                 190

Asn Asp

<210>  10
<211>  100
<212>  PRT
<213>  Homo sapiens

<220>
<221>  Seq 10
<222>  (1)..(100)

<223>  Sequence Description: SEQ ID NO:10:
<400>  10

Met Glu Phe Thr Ala Glu Gln Leu Ser Gln Tyr Asn Gly Thr Asp Glu
1               5                   10                  15

Ser Lys Pro Ile Tyr Val Ala Ile Lys Gly Arg Val Phe Asp Val Thr
            20                  25                  30

Thr Gly Lys Ser Phe Tyr Gly Ser Gly Gly Asp Tyr Ser Met Phe Ala
        35                  40                  45

Gly Lys Asp Ala Ser Arg Ala Leu Gly Lys Met Ser Lys Asn Glu Glu
    50                  55                  60

Asp Val Ser Pro Ser Leu Glu Gly Leu Thr Glu Lys Glu Ile Asn Thr
65                  70                  75                  80

Leu Asn Asp Trp Glu Thr Lys Phe Glu Ala Lys Tyr Pro Val Val Gly
                85                  90                  95

Arg Val Val Ser
            100

## Description

The invention relates to the use of phosphorylated and/or non-phosphorylated membrane associated progesterone receptor component 1 (mPR), in particular of at least one isoform thereof, and to the use of other proteins, in particular isoforms thereof, as diagnostic markers and/or therapeutic targets, to the use of reagents that influence the phosphorylation status of mPR and/or the abundance and/or activity of said other proteins and to the use of an appropriate assay.

Phosphorylation and dephosphorylation of a protein is one of the fundamental activating and deactivating processes in biological systems, in particular with respect to cellular signal transduction processes. Disturbances relating the phosphorylation status (degree of phosphorylation) of a protein are often associated with aberrant biological phenotypes, particularly uncontrolled cell proliferation, that may cause serious diseases, especially cancer.
For instance, breast cancer is one of the most common forms of cancer observed in women in the western civilization, in particular in the United States of America with a predicted number of approximately 215.990 (32%) new cases and with over 40.000 deaths expected in 2005. Consequently there is an increasing demand for a better understanding of molecular events, especially the phosphorylation and dephosphorylation of proteins, underlying cancer and other diseases associated with aberrant biological phenotypes in order to develop improved diagnostic and therapeutic strategies.

Thus it is the object of the present invention to provide a protein that is dependent in vivo on phosphorylation and/or dephosphorylation as diagnostic and/or therapeutic marker, reagents to influence said protein and an appropriate assay allowing for diagnosis and/or therapy of diseases that are related to aberrant biological phenotypes.

To achieve this object, the invention firstly proposes the use of a protein as diagnostic marker and/or therapeutic target as specified in claim 1. Secondly, the invention comprises the use of at least one reagent for diagnosis and/or therapy of diseases associated with aberrant biological phenotypes as depicted in claims 2 and 3. Besides, the invention relates to the use of proteins and reagents for influencing said proteins as depicted in claims 4 to 6. Additionally, the invention relates to the use of an appropriate assay as outlined in claim 7. The wording of all claims (including dependent claims) is hereby incorporated in the description by reference.

According to the invention phosphorylated and/or non-phosphorylated membrane associated progesterone receptor component 1 (mPR), in particular at least one isoform thereof, is used as diagnostic marker and/or therapeutic target for diseases that are associated with aberrant biological phenotypes.

According to a preferred embodiment of the invention mPR, in particular at least one isoform thereof, is at least partially phosphorylated, when used as diagnostic marker and/or therapeutic target for diseases that are associated with aberrant biological phenotypes.

"Membrane associated progesterone receptor component 1 (mPR)" as used herein, comprises the entire mPR protein or any partial sequence thereof, if appropriate synthetically manufactured, in particular by means of genetic engineering, wherein the partial sequence reveals the activity of mPR. Additionally, protein fragments remaining after proteolytic cleavage of the transmembrane domain from the rest of mPR and/or proteins being partially homologous thereto, and their use as medicines, as above are explicitly covered by the term "Membrane associated progesterone receptor component 1 (mPR)".

The term "aberrant biological phenotypes" as used herein comprises all forms of aberrant biological in vivo manifestations, for instance uncontrolled cell proliferation.

"Activity" of proteins as used herein, comprises the enzymatic activity, binding affinity and/or posttranslational activity, in particular phosphorylation.

The term "abundance" as used herein is equivalent to the expression level of proteins, in particular of mPR, being detectable with prior art methods, in particular with the two dimensional ProteoTope® analysis method of the applicant.

Membrane associated progesterone receptor component 1 (mPR, also known as Hpr6.6, or progesterone membrane receptor component 1/PGC1/PGRMC1/PGRC1) is not related to the classical progesterone receptor. mPR has a transmembrane domain N-terminally to a cytochrome b₅ domain that does not interact with heme groups, but is probably involved in steroid binding. Such membrane-associated progesterone receptors are thought to mediate a number of rapid cellular effects not involving changes in gene expression (Lösel, R., Christ, M., Eisen, C., Falkenstein, E., Feuring, M., Meyer, C., Schultz, A. and Wehling, M. (2003) Novel membrane-intrinsic receptors for progesterone and aldosterone. In Watson, C.S. (ed.) The identities of membrane steroid receptors. Kluwer Academic Publishers, Boston, pp. 125-129.). Obviously, they also have the potential to influence gene expression in addition to rapid genome-independent effects. Hpr6.6/mPR reportedly mediate cell death after oxidative damage through a non apoptotic pathway (Hand R. A., Craven R. J., 2003, Hpr6.6 Protein Mediates Cell Death From Oxidative Damage in MCF-7 Human Breast Cancer Cells, J. Cell. Biochem., 90: 534-547). Other results suggest the opposite wherein Hpr6 increases cell survival following chemotherapy (Crudden G., Chitti, . E., Craven R. J., 2005, Hpr6 (heme-1 domain protein) regulates the susceptibility of cancer cells to chemotherapeutic drugs, JPET, 1-23).

The inventors have been the first to prove the evidence of three isoforms of membrane associated progesterone receptor component 1 (mPR) that is described in the following. As a result, it is in particular preferred to use any combinations of these isoforms as diagnostic markers and/or therapeutic targets for diseases that are associated with aberrant biological phenotypes.

In a particular embodiment of the invention the diseases, in particular subgroups thereof, comprise cancer, neurodegenerative diseases, infertility, inflammatory, respiratory and/or pulmonary diseases, wherein cancer, especially breast cancer, is in particular preferred.

According to a especially preferred aspect of the invention phosphorylated and/or non-phosphorylated membrane associated progesterone receptor component 1 (mPR), in particular at least one isoform thereof, may be used as diagnostic marker and/or therapeutic target for subgroups of diseases associated with aberrant biological phenotypes, particularly of cancer, preferably of breast cancer.

For instance, such subgroups can relate to the abundance of at least one protein, in particular of at least one receptor protein, preferably of estrogen receptor. The estrogen receptor (ER) comprises two types of specific nuclear receptors that are known as estrogen receptor α (ERα) and estrogen receptor β (ERβ). Molecular analysis has proven that ERα, like other nuclear receptors, consists of separable domains responsible for DNA binding (DNA binding domain DBD), hormone binding (hormone binding domain HBD) and transcriptional activation domain. The N-terminal activation function (AF-1) of the purified receptor is constitutively active, whereas the activation function located within the C-terminal part (AF-2) requires hormone for its activity.

ERα is found in 50 - 80% of breast tumours and ERα status is essential in making decisions about endocrine therapy with anti-estrogens, which are competitive inhibitors of endogenous estrogens and inhibit mitogenic activity of estrogens in breast cancer. On a molecular basis, they trigger inactive conformation of the ERα, which is then unable to activate transcription (Shiau AK, Barstad D, Loria PM, Cheng L, Kushner PJ, Agard DA, Greene GL. 1998. The structural basis of estrogen receptor/coactivator recognition and the antagonism of this interaction by tamoxifen. Cell. 95:927-37.).

Clinically, a positive ER status (ER⁺, tumour cells showing abundance of ER) correlates with favourable prognostic features, including a lower rate of cell proliferation and histologic evidence of tumour differentiation. In contrast to that, a negative ER status (ER- tumour cells showing no or at least a decreased abundance of ER) corresponds to substantially poorer disease-free and overall survival probability of the patient. ER status is also prognostic for the site of gross metastatic spread. Besides, tumours with high abundance of estrogen receptor (ER⁺ tumours) are more likely to initially manifest clinically apparent metastasis in bone, soft tissue or the reproductive and genital tracks, whereas tumours with low abundance of Estrogen receptor (ER⁻ tumours) more commonly metastasise to brain and liver. Several studies have correlated ERα expression to lower Matrigel invasiveness and reduced metastatic potential of breast cancer cell lines (Platet N, Prevostel C, Derocq D, Joubert D, Rochefort H, Garcia M. 1998. Breast cancer cell invasiveness: correlation with protein kinase C activity and differential regulation by phorbol ester in estrogen receptor-positive and -negative cells. Int. J. Cancer. 75:750-6, and Thompson EW, Paik S, Brunner N, Sommers CL, Zugmaier G, Clarke R, Shima TB, Torri J, Donahue S, Lippman ME, Martin GR, Dixon RB. 1992. Association of increased basement membrane invasiveness with absence of estrogen receptor and expression of vimentin in human breast cancer cell lines. J. Cell. Physiol. 150:534-544.).

Moreover, when ERα-positive cells are implanted in nude mice, tumours appear only in the presence of estrogens and are poorly metastatic as compared to those developed from ERα-negative (ERα⁻) breast cancer cell lines (Price JE, Polyzos A, Zhang RD, Daniels LM. 1990. Tumourigenicity and metastasis of human breast carcinoma cell lines in nude mice. Cancer Res. 50:717-721).

For instance, it was found by the inventors that mPR was significantly more abundant in breast cancer cells showing a negative ER status (ER⁻) compared to breast cancer cells showing a positive ER status (ER⁺). Besides, the inventors have been the first to prove evidence for different degrees of phosphorylation of mPR in breast cancer cells differing in the ER status. The alignment of the phosphorylation status of mPR to subgroups of diseases that are associated with aberrant biological phenotypes, in particular cancer, preferably breast cancer, is advantageous with respect to choice or effectiveness of therapeutic treatments.

For instance, with regard to patients suffering from breast cancer exhibiting a positive ER status tamoxifen is effective in approximately 50% of the cases (Fisher B, Jeong J, Dignam J, Anderson S, Mamounas E, Wickerham DL, and Wolmark N. 2001. Findings from recent National Surgical Adjuvant Breast and Bowel Project adjuvant studies in stage I breast cancer. J Natl Cancer Inst Monogr 30:62-66.).

In addition, the inventors have been the first to detect a wound response signature in breast cancer cells showing a negative ER status (ER⁻) by proteomics, and associated this with a decreased phosphorylation of the membrane associated progesterone receptor component 1 (mPR).

Besides, the present invention comprises the use of at least one reagent for influencing, in particular increasing or inhibiting, the phosphorylation status (degree of phosphorylation) of membrane associated progesterone receptor component 1 (mPR), in particular of at least one isoform thereof, for diagnosis and/or therapy of diseases related to aberrant biological phenotypes.

Furthermore, the present invention encompasses the use of at least one reagent for influencing, in particular increasing or inhibiting, the phosphorylation status (degree of phosphorylation) of membrane associated progesterone receptor component 1 (mPR), in particular of at least one isoform thereof, for the manufacture of a medicament and/or pharmaceutical composition for diagnosis and/or therapy of diseases associated with aberrant biological phenotypes.

In some cases it is desirable that mPR is not completely dephosphorylated. Thus, it is further within the scope of the present invention that said reagent decreases the degree of phosphorylation of mPR to a certain extent. In other cases it may be beneficial to maintain the phosphorylation status of mPR. Therefore, in a further aspect of the invention at least one reagent may be used as diagnostic marker and/or therapeutic target that maintains the phosphorylation status of mPR, in particular at least one isoform thereof.

Furthermore, the invention comprises at least one reagent that is used for diagnosis and/or therapy of diseases associated with aberrant biological phenotypes for influencing, in particular increasing or inhibiting, the interaction of membrane associated progesterone receptor component 1 (mPR), in particular of at least one isoform thereof, with other molecules, especially proteins. Preferably the interaction of at least partially phosphorylated membrane associated progesterone receptor component 1 (mPR) is influenced, in particular increased or inhibited, by said reagent.

In a particular embodiment at least one reagent is used according to the invention that binds to at least partially phosphorylated, preferably completely phosphorylated, mPR in order to prevent the interaction of SH2 and SH3 domains with other molecules, particularly proteins. With respect to molecular details concerning the structure and mechanistic studies of mPR it is referred to the following description.

According to the invention it is in particular advantageous that said reagent is a ligand of mPR that in particular binds to the ligand binding pocket of mPR, or to protein interaction domains of the SH2 and SH3 variety, in order to prevent the interaction of mPR with other molecules, especially proteins, allowing for diagnosis and/or therapy of diseases associated with aberrant biological phenotypes.

Additionally, the present invention comprises the use of proteins, in particular of isoforms thereof, as diagnostic markers and/or therapeutic targets and/or medicines for diseases associated with aberrant biological phenotypes, wherein said proteins are involved, in particular differentially involved, in protein interaction or multi-protein complexes with either phosphorylated or non-phosphorylated membrane associated progesterone receptor component 1 (mPR). With respect to further details, particularly with respect to phosphorylated or non-phosphorylated membrane associated progesterone receptor component 1 (mPR), it is referred to the above description.

It is a further aspect of the present invention that at least one reagent is used for influencing, in particular increasing or inhibiting, the abundance and/or activity of proteins, in particular of isoforms thereof, for diagnosis and/or therapy of diseases associated with aberrant biological phenotypes, wherein said proteins are involved, in particular differentially involved, in protein interaction or multi-protein complexes with either phosphorylated or non-phosphorylated membrane associated progesterone receptor component 1 (mPR). With respect to further details, particularly with respect to phosphorylated or non-phosphorylated membrane associated progesterone receptor component 1 (mPR), it is referred to the above description.

Furthermore, it is within the scope of the present invention that at least one reagent is used for influencing, in particular increasing or inhibiting, the abundance and/or activity of proteins, in particular of isoforms thereof, for the manufacture of a medicament and/or pharmaceutical composition for diagnosis and/or therapy of diseases associated with aberrant biological phenotypes, wherein said proteins are involved, in particular differentially involved, in protein interaction or multi-protein complexes with either phosphorylated or non-phosphorylated membrane associated progesterone receptor component 1 (mPR). With respect to further details, particularly with respect to phosphorylated or non-phosphorylated membrane associated progesterone receptor component 1 (mPR), it is referred to the above description.

The inventors have been the first to discover the association between the state of mPR phosphorylation and disease phenotype. Therefore, a further aspect of the present invention encompasses the use of an assay comprising the determination of the phosphorylation status (degree of phosphorylation) of membrane associated progesterone receptor component 1 (mPR), in particular of at least one isoform thereof, in human samples for diagnosis and/or therapy of diseases related to aberrant biological phenotypes.

According to a particular preferred embodiment of the invention the assay comprises the screening for reagents that are suited for diagnosis and/or therapy of diseases that are associated with aberrant biological phenotypes. Regarding the reagents and diseases it is referred to the above description.

According to a further embodiment of the invention the human samples, preferably microdissected human samples, are derived from a small tissue fraction, particularly from a tumour tissue fraction, advantageously from a breast cancer tissue fraction. The human samples are preferably harvested by biopsy and/or surgical extraction.

Additionally, it is preferred to use the inventive assay for determination of proteins that are involved, in particular differentially involved, in protein interaction or in multi-protein complexes, in particular higher order multi-protein complexes, with either phosphorylated mPR or non-phosphorylated mPR. According to a further embodiment the inventive assay is used for determination of proteins that are colocalized with phosphorylated, preferably hyperphosphorylated, mPR.

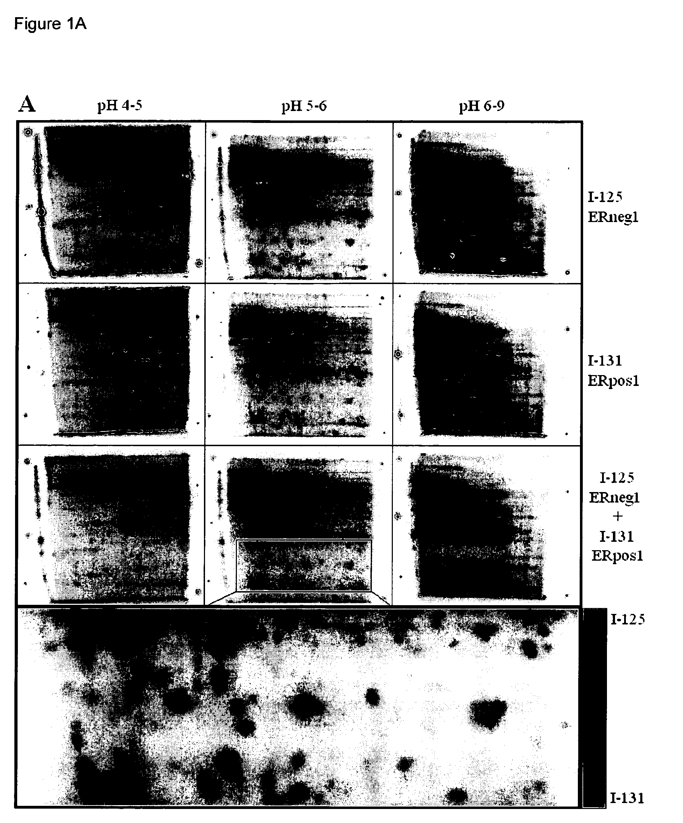

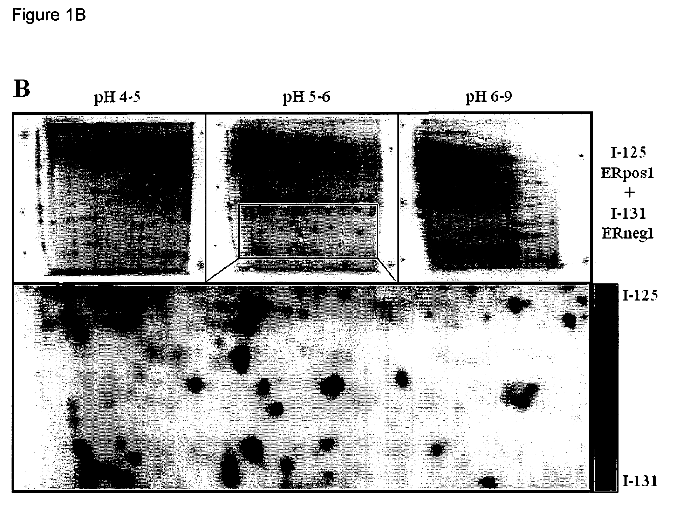

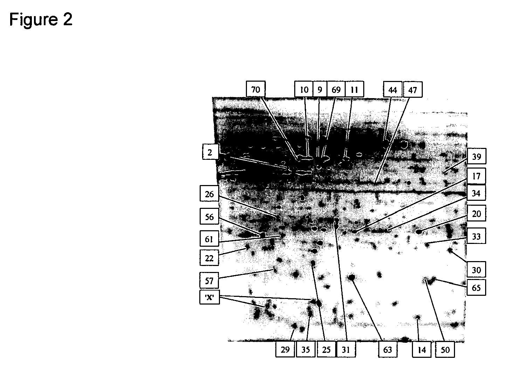

The inventors designed a paired direct comparison strategy by pooling samples derived from tissue sections from large homogenous breast tumours on the basis being either ER⁺ or ER- negative. Eight ER⁺ tumours and eight ER⁻ tumours were used. They were randomly assigned to sub-pools (**table 1**), each sub-pool containing normalised equal amounts of protein from two tumours. For differential analysis, sub-pool ER⁺¹ (containing T378 and T392) was differentially compared to sub-pool ER⁻¹ (containing T433 and T443), ER⁺² was compared to ER⁻², ER⁺³ was compared to ER⁻³, and ER⁺⁴ was compared to ER⁻⁴ (an example of one inverse replicate differential analysis is presented in **figure 1**). Spots were matched across gels, and their intensities were analysed relative to ER status. Synthetic average gel images were constructed by computer (an example of which is given for the pH 5-6 experimental window in **figure 2**). The statistically most significant differential protein spots are preferably identified by mass spectrometry, in particular by MALDI-TOF (**figur**e **3**). In total, proteins from 325 spots were identified by MALDI-TOF PMF with MASCOT scores greater than 70, of which 72 spots represented 16 proteins that were identified in more than one protein spot (**table 2**).

In a further step the inventors performed identification and characterization of proteins revealed by the above described study (table 2), but which had not previously been directly linked to diseases associated with aberrant biological phenotypes, in particular subgroups thereof, especially cancer, preferably breast cancer.

According to a particular embodiment the revealed proteins were identified and characterized regarding their phosphorylation status. According to an especially preferred embodiment among the revealed proteins mPR was characterized by investigating its phosphorylation status in diseases associated with aberrant biological phenotypes, especially cancer, preferably breast cancer. The phosphorylation status (degree of phosphorylation) of mPR, in particular at least one isoform thereof, was investigated in subgroups of breast cancer by the inventors, preferably in subgroups differing in the ER status (ER⁺ versus ER⁻).

In a particular embodiment of the inventive used assay the human samples are subjected to a radioactive labelling, in particular to an inverse radioactive labelling, preferably with iodine isotopes. Preferably an inverse radioactive labelling is performed using ¹²⁵I and ¹³¹I isotopes.

In a further aspect of the invention the assay is based on gel electrophoresis techniques, in particular SDS-PAGE (Sodium Dodecylsulfate Polyacrylamide Gel Elektrophoresis), especially two dimensional PAGE (2D-PAGE), preferably two dimensional SDS-PAGE (2D-SDS-PAGE). According to a particular embodiment the assay is based on 2D-PAGE, in particular using immobilised pH gradients (IPGs) with a pH range preferably over pH 4-9.

According to a further embodiment of the inventive assay the gel electrophoresis techniques, in particular the above mentioned techniques, may be combined with other protein separation methods, particularly methods known to those skilled in the art, in particular chromatography and/or size exclusion.

If appropriate, the above mentioned methods may be combined with detection methods, particularly known to those skilled in the art, in particular antibody detection and/or mass spectrometry.

Since the difference between the mPR isoforms under consideration is due to the presence of phosphate groups, all methods enabling the detection of subtle or extreme differences in the stoichiometry of phosphate or oxygen atoms in proteins are within the scope of the present invention. In this regard, in particular preferred methods may be elemental analysis, measurement of the state of ionisation or differential electrical conductivity. According to a further embodiment methods enabling the measurement of differences in the stable isotope content of proteins, in particular of chemically modified proteins, or degradation products thereof are part of the present invention.

According to a particular aspect of the inventive assay affinity reagents are applied, in particular antibodies. For instance, said affinity reagents, in particular antibodies, may be used in an immunoassay, particularly in an ELISA (Enzyme Linked Immunosorbent Assay), that is preferably part of the inventive assay.

In a further aspect of the invention the assay comprises the application of mass spectrometry, in particular MALDI (Matrix Assisted Laser Desorption/ionization) and/or SELDI (Surface enhanced Laser Desorption/lonization).

Besides, it is within the scope of the inventive assay that resonance techniques, in particular plasma surface resonance, are used.

In some cases it may be advantageous to achieve a separation of proteins, preferably of mPR, in particular by means of one of the above outlined embodiments, before cleaving the proteins.
Such a cleavage step can be performed by applying enzymes, chemicals or other suitable reagents which are known to those skilled in the art.

As an alternative it may be desirable to perform a cleavage step before separation of the peptides, in particular of mPR, obtained by the cleavage step followed preferably by measurements of mPR concerning its abundance and/or degree of phosphorylation.

According to a further embodiment the labelled and in particular separated protein spots are visualized by imaging techniques, for instance by the Proteo Tope® imaging technique of the applicant.

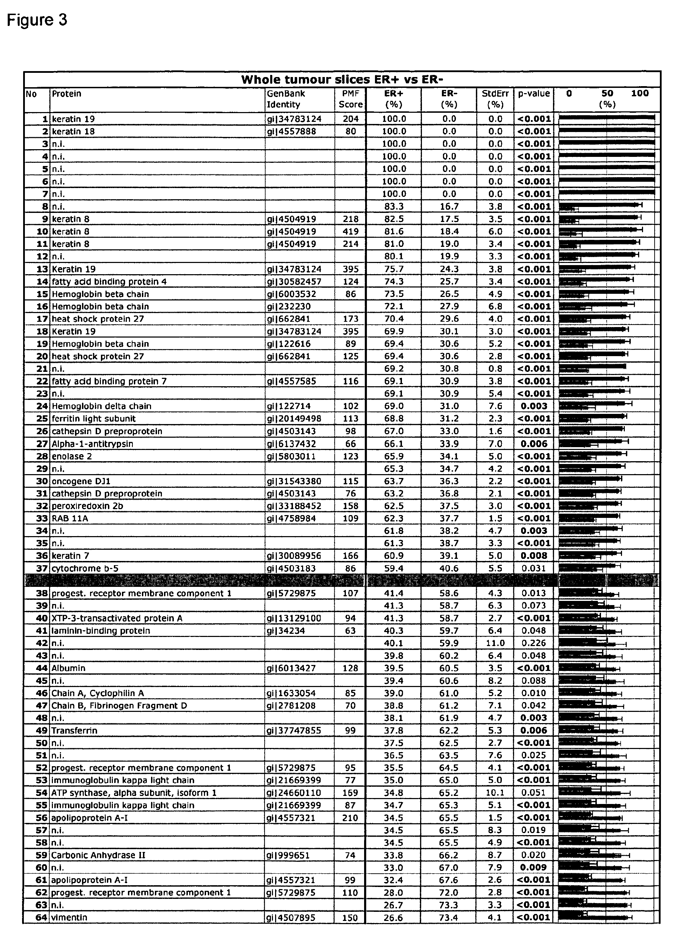

Membrane associated progesterone receptor component 1 mPR was identified by the inventors from three spots (**table 2**) that formed an approximately equidistant chain in the pH 4-5 IPG, two of which (**figure 3**: spots 52, and 62) were significantly more abundant in cancers with a negative ER status (ER⁻). Furthermore, these more acidic spots exhibited slightly retarded migration in SDS-PAGE (Sodium Dodecylsulfate Polyacrylamide Gel Elektrophoresis), consistent with possible phosphorylation differences between the spots. To test the hypothesis that these quantitative differences were due to altered isoelectric points of the protein caused by differential phosphorylation in the spots, and that phosphorylation therefore may differentially affect the intracellular localisation of mPR between the test tissues, the inventors devised a phosphatase treatment regime using shrimp alkaline phosphatase (SAP). Whole cell native protein extracts from several patients were pooled and incubated with either phosphatase buffer containing SAP (+SAP), or mock incubated under identical conditions with the addition of phosphatase inhibitors but without SAP (-SAP). A raw extract that was not incubated at all prior to protein denaturation (raw) was also included in the analysis as a reference control.

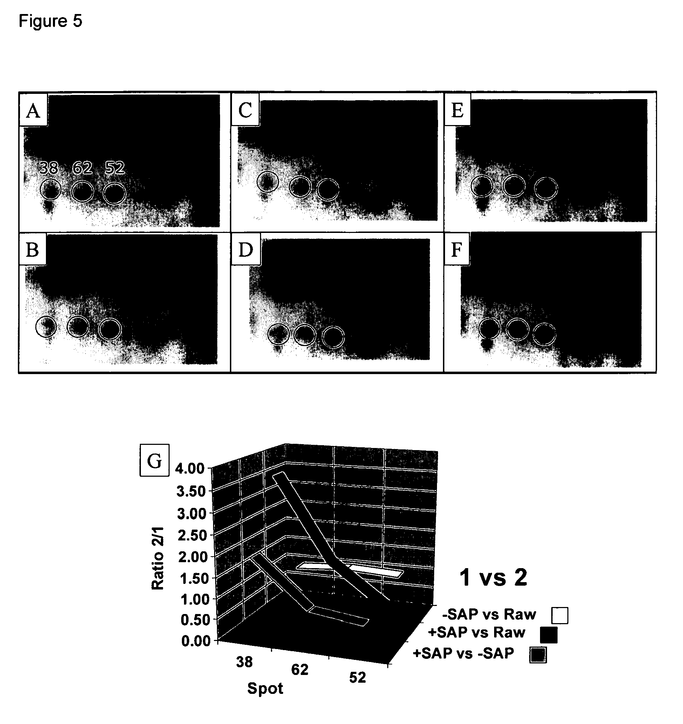

For instance, the samples were analysed by the inventors pairwise against each other by ProteoTope® imaging after inverse radioactive labelling with ¹²⁵I and ¹³¹I, and separation by daisy chain 2D-PAGE. The portions of the part of the inverse replicate gels containing the mPR spots are shown in **figure 5**. Panel A, being ¹²⁵I-labelled +SAP sample (blue) and ¹³¹I-labelled mock -SAP sample (orange) shows a discernable preponderance of ¹³¹I for the most acidic spot (spot 38). By contrast, the most basic of the spots (spot 52) exhibited a slight preponderance of ¹²⁵I. Importantly, these small differences in relative signal intensity were reproducibly detected in the inverse replicate labelled experiment of Panel B, where spot 38 also shows a discernable preponderance of ¹²⁵I and spot 52 of ¹³¹I. Panels B and C compared the phosphatase treated samples against the untreated raw extract. The same trend was observed, however the magnitude of the differences was slightly higher, the difference being possibly due to experimental error. By contrast, when the mock treatment was compared to the raw extract in panels E and F, the ratios between both samples approximated 50%. Thus, the difference in intensity of this spot was not due to the incubation, but rather due to the presence of phosphatase in the incubation. The averaged quantified results from both inverse replicate dual image gels for each sample comparison are presented graphically in Panel G. This result strongly demonstrates that the most acidic spots can be dephosphorylated, whereupon they migrate to one of the more basic spots. Taken together with the results of **figure 3** for these three protein spots, this provided evidence that mPR is probably more highly phosphorylated in ER⁺ than ER⁻ tumours.

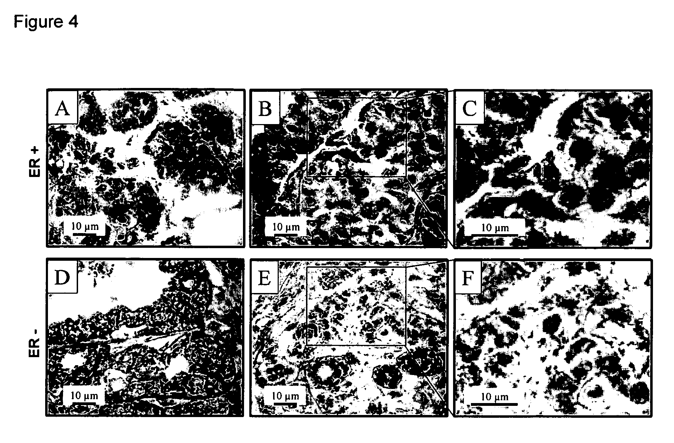

Therefore, the inventors provided evidence that the population of mPR molecules is more highly phosphorylated in ER⁺ tumours than ER⁻ tumours. Consequently, this is the first *de-novo* demonstration of a phosphorylation difference from primary tumours by discovery proteomics without the use of cell culture. Interestingly, this phosphorylation pattern corresponds to the presence of punctuated localised concentration of mPR in extra-nuclear regions of ER⁻ cells (**figure 4**).

The inventors further examined mPR for potential protein motifs in table 3 for mPR (SwissProt entry 000264). For a protein of just 194 amino acids (21.5 kDa), in addition to the cytochrome b₅ domain, a plurality of predicted short motifs concerned with protein interactions and signal transduction molecules is present, including two SH2 domains, an SH3 domain, a tyrosine kinase site, two CK2 sites, and consensus binding sites for ERK1 and PDK1. Furthermore, these are all on the surface of the folded protein. Although not all of these sites may be biologically relevant, this nevertheless strongly suggests that mPR may be able to function as an adaptor molecule in signal transduction processes. The inventors noted that both of the CK2 sites have been detected as phosphoserine peptides in mPR from HeLa cell nuclear extracts (Beausoleil SA, Jedrychowski M, Schwartz D, Elias JE, Villen J, Li J, Cohn MA, Cantley LC, Gygi SP. 2004. Large-scale characterization of HeLa cell nuclear phosphoproteins. Proc Natl Acad Sci U S A. 101:12130-5; Table 3).

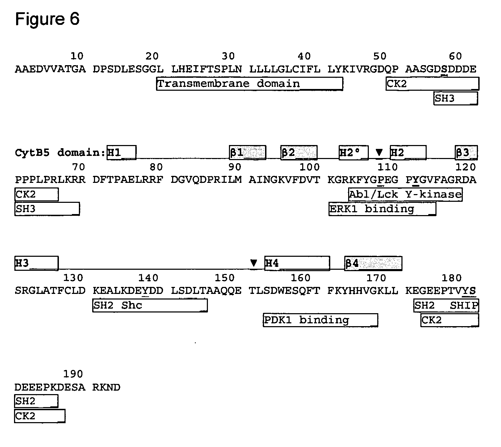

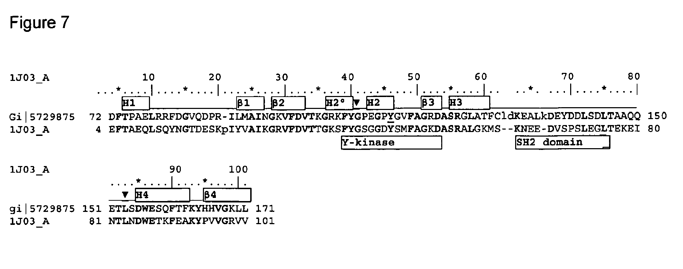

Considerable information is available concerning the structure and homologous conservation of the cytochrome b₅ domain proteins. Indeed the predicted sites from **table 3** exhibit high conservation, sometimes from yeast to mammals. The inventors aligned the conserved structural beta strands and helical elements to the sequence of mPR, including the predicted protein motifs from **table 3** (SEQ ID NO:1-8). Remarkably, the two CK2 sites flanked the conserved cytochrome b₅ domain (SEQ ID NO:10), and each of these overlapped with a predicted SH2 and SH3 domain respectively (**figure 6**, SEQ ID NO:9). This immediately suggests the possibility that CK2 could negatively regulate protein interactions mediated by these domains, and/or that phosphorylation by CK2 could allosterically affect the conformation of the ligand binding domain. To assess this, the inventors examined two available protein structures: the bovine cyt-B5 structure (Durley and Mathews, 1996) and the cytochrome b₅-domain *Arabidopsis* protein "Putative Steroid-Binding Protein" (Genbank Accession GI:40889041) for which an NMR structure has been submitted by Suzuki et al. in 2002 to the Research Collaboratory for Structural Bioinformatics (RCSB: http://www.rcsb.org/pdb/) Protein Data Base (PDB) with PDB Accession 1J03). Alignment of the cytochrome b₅ domain of the *Arabidopsis* protein with mPR confirm that the major structural elements are conserved, importantly, including the approximate number and partial identity of amino acids in the putative SH2 domain between Helices 3 and 4 (**figure 7**). According to the inventors these proteins are probably structurally homologous over this domain, and that the NMR structure of 1 J03 can validly be superposed to the amino acid backbone of mPR.

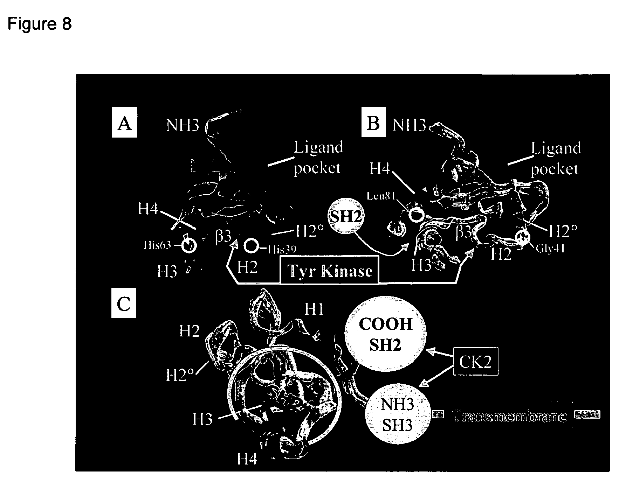

The structures of these two proteins are presented graphically in figure 8, showing the structural motif labelling nomenclature from **figure 6** according to the mPR amino acids in SwissProt Accession 000264. Using the aligned domain topology of these two protein structures, the inventors noted that the N-terminal and C-terminal regions of both cytochrome b₅ domains were on the opposite side of the protein than the ligand binding domain. Therefore the predicted mPR N-terminal SH3 site and CK2 site centred on residues P62 and S62 respectively (**table 3**) are most probably on the surface of the related mPR protein directed away from the ligand binding pocket. Similarly, the predicted SH2 site and CK2 consensus site centred on Y179 and S180 respectively are also probably located away from the ligand binding site. This suggests that ligand binding of mPR could occur simultaneously with protein interactions through these sites. The inventors also observed that the predicted Shc consensus-like SH2 domain was inserted into a loop between conserved cytochrome b₅ domain helices 3 and 4 in the superposed structures. In the crystal structure of bovine cyt-b5, these two helices are joined by a short linker at the rim of the ligand binding domain. In the plant protein the amino acids between helices 3 and 4, which exhibit marked amino acid homology to the predicted SH2 motif of mPR, fold to the side away from the ligand pocket, leaving the ligand binding domain accessible.
Interestingly, structural alignment of mPR with particularly the plant protein also revealed that P108 at the centre of the putative ERK1 binding site (**table 3**) was exposed at the surface of the protein immediately N-terminal to helix 2° (**figure 6**), while T160 at the centre of the putative PDK1 kinase binding site was also exposed on the surface of helix 4. In fact, the P108 putative ERK1 binding site is situated on the lip of the binding pocket adjacent to the position where a cyt-B5 histidine interacts with the heme ligand, and this proline is conserved in related proteins from yeast to mammals. Therefore the putative ERK1 binding could conceivably be inhibited by recruitment of the ligand to the binding pocket. The kinase PDK1 (3-phosphoinositide-dependent protein kinase-1) phosphorylates and activates kinases that are regulated by PI 3-kinase, which in turn regulate cellular metabolism, growth, proliferation and survival, all of which may be related to the biology of ER- tumours. The functional relevance of these putative kinase binding sites, and the putative involvement of these kinases in the breast cancer-associated biology of mPR, merit further scrutiny.

In view of the above mentioned observations a model for the cellular function of mPR in breast cancer was considered by the inventors. As stated above, the preponderance of predicted protein motifs associated with signal transduction in the 21.5 kDa protein shows that the protein indeed functions as a signalling adaptor molecule, and that the activity may be progesterone-responsive, or responsive to other ligands, such as heme. An interplay between ligands is a possible mechanism of regulation which offers potential avenues of pharmaceutical intervention. Strikingly, the spatial juxtaposition of the SH2 and SH3 motifs in **figure 8** would induce intimate local concentration of potential interacting proteins relative to each other, such as possibly kinases and their substrates, potentially greatly facilitating enzymatic activities and biological actions.

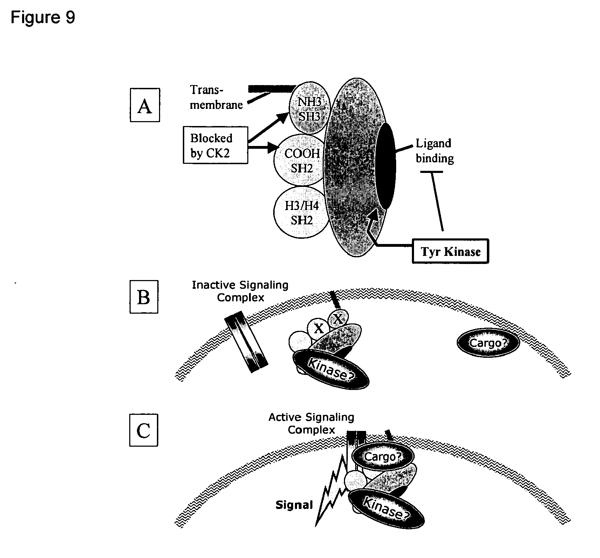

This provides explanation for the mode of action of mPR. The protein is schematically represented in **figure 9A** as a ligand binding signalling adaptor protein. In the ER⁺ state, one or more of the N-terminal SH3 domain and C-terminal SH2 domain are phosphorylated by CK2 or related kinases, and do not interact with certain other proteins. Since the C-terminal CK2 site is more highly evolutionarily conserved, and overlaps exactly with the corresponding SH2 domain, it is the more likely of these motifs to be functionally regulated by CK2, however both sites have been observed to be phosphorylated. However in the ER⁻ state the CK2 site/s is/are dephosphorylated, leading to relocation of mPR to specific subcellular compartments, as observed in **figure 4**. The SH2 domain between helices 3 and 4 of the cytochrome b5 domain may interact with other proteins in both cell types. However interactions through this domain are regulated by ligand binding, tyrosine phosphorylation of the Abl/Lck-like site at Y112, and/or binding of either ERK1 and/or PDK1 to their respective sites at the ligand pocket. Figure 9B depicts one possible model for mPR in ER⁺ cancers. The protein exists predominantly in the state phosphorylated by the constitutive kinase CK2, preventing the predicted protein interactions through the SH3 and SH2 domains that flank the cytochrome b₅ domain. In **figure 9C** the CK2 sites are dephosphorylated in ER- cancers, leading to interaction with other proteins, sub-cellular relocalisation, and activation of signal transduction. The present results reveal significant differences in mPR between ER⁺ and ER⁻ tumours for the first time, and further reveal that this protein is potentially a potent target for in particular anticancer therapy of ER⁻ tumours.

The tables and figures relate to the following:

### Table 1: pooling design for ER⁺ vs ER⁻ cryogenic whole tumour sections

Individual tumours are designated by their tumour bank T registration numbers. Experimental, clinical and histopathological parameters are listed. Eight ER⁺ and eight ER⁻ tumours are grouped into four pools of two tumours each as indicated. Clinical data comprise: tumor status ranging from pT1 (tumor 2 cm or smaller in greatest dimension) to pT3 (tumor >5cm); Lymph node status from pN0 (no regional lymphnode metastasis) to pN3 (metastasis to ipsilateral internal mammary lymph nodes(s)) and pNx (regional lymph node cannot be assessed); tumour grade from 2 (moderately differentiated) to 3 (poorly differentiated); Histopathological data for ER and PR (0: undetectable, 1-3: weakly positive, 4-7: moderately positive, 8-12: highly positive); and HER2/neustatus (0=negative, positive +1 to +3). Ages for each patient are given in years.

### Table 2: protein spots that contained multiple identifications of individual proteins as gene products

The protein name and number of spots are indicated in the column headings. Approximate estimates for the experimentally observed isoelectric point (PI) and molecular weight (MW) are given for each spot, as are Genbank accession numbers and PMF scores, the nomenclature conventions for which follow Figure 3.

### Table 3:

Protein motifs contained in mPR (SwissProt entry 000264). 1. The position within the mPR sequence of the amino acid at the center of the predicted motif is given in the column. 2. The amino acid from column 1 shown in the context of the flanking amino acids that belong to the sequence motif. 3. The type of motif predicted to be present in columns 1 and 2. "Acidophilic S/T Kinase" = acidophilic type serine/threonine kinase. "SH3" = Src homology 3 domain, "Kinase binding" = predicted binding site for a protein kinase. "Tyr-Kinase" = consensus tyrosine kinase phosphorylation site. "SH2" = Src Homology 2 domain, Column 4 gives the specific type of consensus motif from column 3.

### Figure 1: 54 cm differential ProteoTope® analysis

The panels show actual images from an inverse replicate labelled ProteoTope® experiment for one sample pair. (**A**) Analysis of pooled sample ER⁺¹ (ERpos1) from table 1 labelled with I-125, differentially compared with pooled sample ER⁻¹ (ERneg1) labelled with I-131. The lower panels show the signal detected for each isotope, depicted in false spectral colour. The signals for each isotope have been normalised against each other for total relative intensity in the upper dual channel images, where the signal for I-125 is blue, the signal for I-131 is orange, and equal amounts of both signals produces grey or black signal. Two pure sources each of I-131 and I-125, as well as a 50% mixture of both isotopes, are measured on round 2 mm pieces of filter paper placed next to each gel as imaging controls. Cross talk between the signals from each isotope is <1%. The pH ranges of the 18 cm IPGs used for serial IEF are indicated above the panels, and the radioactive iodine isotope signals depicted in each panel are indicated on the right. In this experiment all iodination reactions were performed on 60 µg protein. In the examples shown, the I-125 is signal is systematically stronger in all gels (compare lower panels for individual isotopes). (**B**). The top panels show the inverse replicate experiment of A, where sample ER⁺¹ is labelled with I-131, and sample ER⁻¹ is labelled with I-125. The bottom panel shows an enlarged portion of a gel image, as indicated. Similar gels were produced for all corresponding differential analyses depicted in table 1.

### Figure 2: Typical example of a synthetic average composite gel of the pH 5-6 analysis, showing spots matched across all gels in the study in this pH range from Figure 1

The average ER⁺ signal is indicated as blue, the average ER⁻ signal is indicated as orange, and equal intensities of both signals give grey or black pixels. Spot numbers correspond to Figure 3. Some orange or blue spots that are not numbered (e.g., those labelled 'X') were not visible on preparative silver stained tracer gels, and were omitted from the analysis. This image was generated with the GREG software. Labels were added manually.

### Figure 3: Protein spot quantification and identifications for breast cancer samples (whole tumour slices) comparing ER positive and ER negative samples

n.i. = not identified. Genbank Identities are from the NCBI data base version of Apr 4, 2004. MALDI-TOF peptide mass fingerprinting (PMF) scores are from MASCOT. The average spot fraction for ER⁺ and ER⁻ are given as percent of the normalised total spot volume for each spot (=(ER⁺ x 100%)/(ER⁺ + ER⁻)) across all patient pools based on two colour ProteoTope® analysis for the indicated most significant protein spots. These values were obtained using a least square fit for a model based on all replicates and attributing pool variability as a random effect. The t-test p-value for this model is also given. P-values < 0.01 are bold, and p-values < 0.001 are designated as such. The bars at the right depict average percent abundance of each protein across the ER⁺ (dark blue) and ER⁻ (light orange) pools as indicated above the column with bars (0% - 50% - 100%). Error bars show standard error of means. Protein spots between numbers 37 and 38 (indicated by a grey field) are not presented, having failed to meet selection criteria of either abundance difference ratio of 1.5 or significance at the 5% level.

### Figure 4: mPR immune histochemistry in ER⁺ and ER⁻ tumours

The rabbit polyclonal anti-mPR-specific signal (green) is associated with diffuse cytoplasmic staining in ER⁺ tumours (**A-C**), whereas anti-mPR signal exhibits increased localised concentration to specific extra-nuclear sub-cellular locations in ER⁻ cells (**D-F**). The dark purple colour is hematoxylin counter-staining of nuclear chromatin. The 10 µm scale bar is shown in each panel. A and B show the mPR staining pattern of two different tumours, while C shows an enlargement of the framed region from B, as indicated. The same relationship applies to D,E and F.

### Figure 5: Differential quantification of phosphatase treated and control samples

(**A-F**) Inverse replicate ProteoTope® images of the gel region containing three spots of mPR: spots 38, 52, and 62 from Figure 3. Image conventions follow Figure 1. (**A**) The phosphatase treated sample (+SAP) is labelled with I-125 (blue colour), and the mock incubation control (-SAP) is labelled with I-131 (orange colour). Spot numbers are indicated, and are applicable to all panels. (**B**) The inverse replicate experiment to A. (**C**) I-125 labelled +SAP is analysed I-131 labelled untreated raw control sample (raw). (**D**) The inverse replicate experiment to C. (**E**) 1-125 labelled - SAP is analysed against I-131 labelled raw control. (**F**) The inverse replicate experiment to E. (**G**) Quantification of the differential ratio of of signal intensities from sample 2/sample 1 for each of the spots from the gels shown in A-F. The identity of sample 1 and sample 2 for each comparison are shown at the right hand side of the panel, with corresponding colour coding. The ratio of signal for control and treated samples increases in a phosphatase-dependent manner, consistent with spots 38 and 62 representing phosphorylated isoforms of spot 52.

### Figure 6: Position of structural motifs conserved for the mPR Cyt-b₅ domain

Amino acids of the cyt-b₅ (cytochrome b₅) domain of mPR numbered according to SwissProt Accession 000264 (SEQ ID NO: 10) are boxed and shaded. Helices (H1-H4) and beta strands (β1- β4) are as published (Mifsud and Bateman, 2002), except helix H2°, which was added by the authors according to the crystal structure of bovine cyt-b₅. Triangles above G107 and L152 represent positions in the structure where corresponding histidine residues interact with the ligand heme group in the structure of cyt-b₅. Amino acids predicted to be phosphorylated at consensus kinase sites are underlined. In addition to the motif predictions from table 3, the predicted transmembrane domain from SwissProt is indicated between amino acids 20-42. Figure 6 shows the sequence of membrane associated progesterone receptor component 1 (mPR) (SEQ ID NO: 9).

### Figure 7:

Sequence alignment of the cytochrome b₅ domains of mPR (gi|5729875) and the *Arabidopsis* Putative Steroid Binding Protein 1J03_A. Amino acid numbering is that of 1J03_A. Conserved amino acids are in bold print. Other notation follows the convention of Figure 6 (the mPR sequences designated by SwissProt Accession 000264, and NCBI Gene-Bank Identifier gi|5729875 represent the same protein, but differ by the presence or absence of the N-terminal initiator methionine).

### Figure 8: Structural modelling of the cytochrome b₅ domain of mPR, and flanking motifs, indicating structural domains following Figure 6

Structures were manipulated using the RasMol program. (**A**). The crystal structure of bovine cyt-b₅ (PDB Accession 1CYO). The ligand binding pocket is indicated, and the orthologous position of the predicted tyrosine kinase phosphate acceptor from mPR (Figure 6) is indicated in A and B. The position of ligand-interacting His39 and His63 are also indicated, as are the corresponding Gly41 and Leu81 in B. (**B**). The NMR structure of *Arabidopsis* "Putative Steroid-Binding Protein" (PDB Accession 1 J03_A) shown looking down onto the ligand binding pocket in similar orientation to structure in A. (**C**). The structure from B is rotated to view the opposite surface, showing the inferred adjacent locations of the src homology domain structural motifs predicted for mPR. The position where the predicted SH3 domain at the N-terminal, and SH2 domain at the C-terminal of the superposed cytochrome b₅ domain of mPR are schematically indicated, as are the helix-3/helix-4 SH2 domain and the N-terminal transmembrane region.

### Figure 9: Hypothetical model for mPR function as an adapter molecule in signalling.

(**A**) Schematic representation of putative mPR functional modules (Figure 6 and Figure 7). (**B**) The situation in ER⁺ tumours. One or more CK2 sites are phosphorylated ("x"), inactivating the interaction with other proteins (eg membrane receptors and "Cargo?") through the respective SH3 and/or SH2 domains N- and C-terminal to the Cyt-b₅ domain. One or more kinases may be bound to mPR, such as ERK1 or PDK1 to their respective predicted sites, or a tyrosine-phosphorylated signal transduction-effecting molecule such as a tyrosine kinase or phosphatase to probably the Helix3-Helix4 SH2 domain. (C) The situation in ER⁻ tumours. The CK2 site phosphorylation state is reduced, permitting interaction with cargo proteins and signaling receptors to form active signal transduction complexes. Possibly, ligand binding could affect the situation in C. This association is a further part of the present invention.

Further advantages, features and possible uses of the invention are described below by means of the exemplary embodiments with reference to the above described tables and figures. In this connection, the various features may in each case be implemented on their own or in combination with one another.

### Materials and Methods

Patients and tissue samples. Primary breast cancer specimens were obtained with informed consent from patients, who were treated at the Department of Gynecology and Obstetrics, University Hospital Tübingen (Ethikkommission Med. Fakultät AZ.266/98). Samples were characterized and collected by an experienced pathologist. After removal of breast tumour from the patient, the tissue samples were embedded in O.C.T. compound (Leica), then snap frozen in liquid nitrogen within 15 minutes of tumour removal, and stored at -196°C in a tumour tissue bank. Sample collection was approved by an ethics committee and by the patient. Tumour data were stored in an Oracle-based database according to practices approved by the Institute of Electrical and Electronics Standards Association (IEEE-SA). Clinical information was obtained from medical records and each tumour was diagnosed by a pathologist, according to histopathological subtype and grade. The tissue quality of each tumour was verified by measuring RNA integrity from one or more slices with an Agilent 2001 Bioanalyser. Tumours lacking sharply distinct 18S and 28S ribosomal RNA bands were excluded from the study. ER, PR and HER-2/neu status for each tumour were routinely determined by immunohistochemistry.

### Preparation of cryosections

Tumour samples were selected using the database, removed from the tissue bank on frozen CO₂ and transferred to a cryotome (Leica) at a temperature of -23°C. Cryogenic sections (10 µm) were subsequently sliced, placed on SuperFrost+-slides (Multimed) and stored at -80°C until further use. For immunopathologic characterisation by an experienced pathologist one section was stained with hematoxilin/eosin.

### Proteomics analysis

ProteoTope® analysis was performed essentially as described (Schrattenholz A, Wozny W, Klemm M, Schroer K, Stegmann W, Cahill MA. 2005. Differential and quantitative molecular analysis of ischemia complexity reduction by isotopic labelling of proteins using a neural embryonic stem cell model. J Neurol Sci. 229-230:261-7). Frozen tumour sections of 10 µm were lysed directly into SDS buffer, separately iodinated in inverse replicated with each of I-125 and I-131, and separated by 54 cm daisy chain IEF-IPG after sample pooling as described in Table 1. Aliquots of each sample were each iodinated by either ¹²⁵I or ¹³¹I, respectively, using approximately 6 MBq of each isotope per 3.6 µg pooled sample aliquot under identical chemical conditions in a reaction volume of 25 µL by the iodogen method as described. Radioactive iodine was purchased from Amersham Biosciences (Freiburg). 2D-PAGE was performed using 18 cm commercial immobilised pH gradients (IPGs) in serial 54 cm IPG-IEF over pH 4-9 (pH 4-5; pH 5-6; pH 6-9) that were run in the SDS-PAGE dimension as 3 x 18 cm IPGs in a Hoefer ISO-DALT.

### Shrimp Alkaline Phosphatase (SAP) analysis.

Cryogenic slices from 6 patients (30 slices T433, 40 slices T443, 40 slices T469, 40 slices T470, 35 slices T623, 30 slices T640) were each extracted with 200 µL aliquots of SAP-dephosphorylation buffer (50 mM Tris pH 8.5, 5mM MgCl₂, 0.25% CHAPS, supplemented with 1x EDTA-free Complete protease inhibitor cocktail from Roche). This precooled buffer was added directly on ice to the frozen slices in eppendof tubes and the tissue was mechanically homogenised using a plastic pellet pestle. Tubes were vortexed and incubated for 30 min at 4°C, followed by centrifugation for 15 min at 14 000 x G at 4°C. Supernatants were collected together and the protein concentration was assayed by the BCA method as described. The yield was approximately 4 mg of protein. 30 Units of SAP in 30 µL were added into 800 µg of protein in 400 µL of in SAP-Dephosphorylation buffer, followed by mixing and incubation for 16 h at 37°C. In parallel a mock incubation control was performed on 800 µg of protein in the same buffer without the addition of SAP, and containing the following phosphatase inhibitors: activated vanadate, sodium fluoride, and sodium glycerophosphate at final concentrations of 1mM, 5mM, and 5mM respectively. The incubation was performed in parallel at 37°C for 16 h. Following incubation the proteins were frozen at -80°C. A non-incubated raw lysate control containing 800 µg of protein in 400 µl of SAP buffer was frozen at -80°C without additions or incubation. Frozen protein mixtures were thawed, precipitated, and resuspended at 1µg/µl in boiling 0.1 M Tris, 2% SDS, pH8.5. 60 µg of protein were then used for iodination with each of I-125 or I-131 as described. Differential inverse replicate ProteoTope analysis was as described above for 54 cm daisy chain IPG-IEF after rehydration loading overnight to the pH 5-6 IPG.

### Protein Identification by Mass Spectrometry

Protein identification is based on different mass spectrometric methods: an automated procedure that allows a very quick and reliable identification of higher abundant proteins (peptide mass fingerprinting with MALDI-TOF-MS) but also allows the identification of very low abundant proteins with more time consuming procedures (LC-ESI-IonTrap-MS/MS, or MALDI TOF-TOF). Briefly, gel plugs of selected protein spots are excised and the proteins contained in the gel plugs are digested using trypsin. The resulting solution is analysed first with a high throughput peptide mass fingerprint procedure based on MALDI-TOF-MS. For those spots where only ambiguous identification was achieved, a fragment ion analysis based on MALDI TOF-TOF or LC-ESI-lonTrap-MS/MS was added. A detailed description of typical MALDI-TOF-MS procedures has been published (Vogt JA, Schroer K, Holzer K, Hunzinger C, Klemm M, Biefang-Arndt K, Schillo S, Cahill MA, Schrattenholz A, Matthies H, Stegmann W. 2003. Protein abundance quantification in embryonic stem cells using incomplete metabolic labelling with 15N amino acids, matrix-assisted laser desorption/ionisation time-of-flight mass spectrometry, and analysis of relative isotopologue abundances of peptides. Rapid Communications in Mass Spectrometry, 2003;17:1273-1282).

### Database Searching

For the identification of the proteins the peptide masses extracted from the mass spectra were searched against the NCBI non-redundant protein database (www.ncbi.nlm.nih.gov) using MASCOT software version 1.9 (Matrix Science, London, detailed description can be found at http://www.matrixscience.com/).

**Table 1**

| **Experimental variable designation** | **Pools** | **Tumor number** | **RNA Quality** | **Tumor status** | **Lymph node status** | **Grade** | **ER status** | **PR status** | **Her2/ neu-Status** | **Age of patient** |
|---|---|---|---|---|---|---|---|---|---|---|
| **ER+** | **ER+1** | T378 | ok | 2 | 0 | 2 | 12 | 4 | 0 | 75 |
| | | T392 | ok | 2 | 0 | 2 | 12 | 2 | 2* | 61 |
| | **ER+2** | T460 | ok | 2 | 0 | 2 | 4 | 8 | 3 | 79 |
| | | T464 | ok | 2 | 0 | 2 | 12 | 8 | 0 | 50 |
| | **ER+3** | T288 | ok | 2 | 0 | 2 | 12 | 4 | 1 | 76 |
| | | T711 | ok | 4 | 2 | 3 | 8 | 6 | 0 | 65 |
| | **ER+4** | T712 | ok | 2 | 1 | 2 | 9 | 4 | 0 | 58 |
| | | T425 | ok | 2 | 1 | 2 | 12 | 0 | 0 | 78 |
| | | | | | | | | | | |
| **ER-** | **ER-1** | T433 | ok | 2 | 0 | 2 | 0 | 0 | 1 | 42 |
| | | T443 | ok | 2 | 1 | 2 | 0 | 12 | 0 | 46 |
| | **ER-2** | T469 | ok | 1 | 0 | 2-3 | 0 | 0 | 3 | 50 |
| | | T470 | ok | 2 | 1 | 2 | 0 | 0 | 0 | 39 |
| | **ER-3** | T531 | ok | 2 | 0 | 2 | 0 | 0 | 0 | 58 |
| | | T558 | ok | 2 | 0 | 2-3 | 0 | 0 | 0 | 62 |
| | **ER-4** | T623 | ok | 1 | x | 2-3 | 0 | 0 | 1 | 42 |
| | | T640 | ok | 2 | 0 | 3 | 0 | 0 | 3 | 62 |

| | | | | | | | | | | |
|---|---|---|---|---|---|---|---|---|---|---|
| * : no Gene Amplification detected x: pNx regional lymphnodes cannot be assessed | | | | | | | | | | |

**Table 2**

| **Protein Name** | **Number of Spots** | **Experimental** | | **Genbank AccNo** | **PMF Score** |
|---|---|---|---|---|---|
| | | **PI** | **MW** | | |
| Albumin | 19 spots | 5.8 | 73000 | gi\|23307793 | 87 |
| | | 5.3 | 71000 | gi\|6013427 | 54 |
| | | 5.3 | 67000 | | 72 |
| | | 5.3 | 66000 | | 58 |
| | | 5.4 | 83000 | | 75 |
| | | 5.4 | 72000 | | 93 |
| | | 5.4 | 72000 | | 96 |
| | | 5.4 | 62000 | | 85 |
| | | 5.4 | 60000 | | 70 |
| | | 5.5 | 72000 | | 112 |
| | | 5.5 | 71000 | | 158 |
| | | 5.5 | 70000 | | 86 |
| | | 5.6 | 71000 | | 136 |
| | | 5.6 | 71000 | | 144 |
| | | 5.6 | 50000 | | 63 |
| | | 5.7 | 104000 | | 94 |
| | | 5.7 | 71000 | | 88 |
| | | 5.7 | 71000 | | 90 |
| | | 5.7 | 71000 | | 128 |
| ATP synthase | 2 spots | 6.7 | 55000 | gi\|24660110 | 83 |
| | | 6.9 | 57000 | | 169 |
| Carbonic Ahydrase II | 2 Spots | 6.5 | 27000 | gi\|1633065 | 78 |
| | | 6.7 | 26000 | gi\|999651 | 74 |
| Cyclophilin A | 3 Spots | 6.8 | 16000 | gi\|1633054 | 87 |
| | | 7.4 | 15000 | | 81 |
| | | 7.0 | 16000 | | 85 |
| Fibrinogen beta | 5 spots | 6.1 | 59000 | gi\|399492 | 79 |
| | | 6.1 | 57000 | | 85 |
| | | 6.8 | 55000 | | 66 |
| | | 7.1 | 57000 | | 88 |
| | | 5.6 | 40000 | gi\|2781208 | 70 |
| HSP27 | 2 spots | 5.4 | 26000 | gi\|662841 | 123 |
| | | 5.5 | 26000 | | 125 |
| Keratin 7 | 3 spots | 5.2 | 55000 | gi\|30089956 | 97 |
| | | 5.2 | 54000 | | 112 |
| | | 5.3 | 56000 | | 273 |
| Keratin 8 | 4 spots | 5.3 | 56000 | gi\|39645331 | 154 |
| | | 5.1 | 49000 | gi\|4504919 | 102 |
| | | 5.2 | 49000 | | 215 |
| | | 5.4 | 55000 | | 419 |
| Keratin 9 | 4 spots | 5.1 | 67000 | gi\|435476 | 217 |
| | | 5.1 | 66000 | | 141 |
| | | 5.1 | 16000 | | 132 |
| | | 5.1 | 12000 | gi\|4557705 | 72 |
| Keratin 19 | 4 spots | 5.0 | 43000 | gi\|34783124 | 407 |
| | | 5.0 | 42000 | | 395 |
| | | 5.0 | 42000 | | 424 |
| | | 4.9 | 41000 | | 204 |
| Alpha I antitrypsin | 14 spots | 4.5 | 60000 | gi\|1942629 | 126 |
| | | 4.8 | 62000 | | 103 |
| | | 4.8 | 54000 | | 97 |
| | | 4.8 | 52000 | | 71 |
| | | 4.8 | 51000 | | 93 |
| | | 4.8 | 50000 | | 80 |
| | | 4.9 | 63000 | | 186 |
| | | 4.9 | 50000 | | 97 |
| | | 5.0 | 61000 | | 232 |
| | | 5.0 | 60000 | | 254 |
| | | 5.0 | 49000 | | 149 |
| | | 5.0 | 61000 | | 130 |
| | | 5.1 | 60000 | | 133 |
| | | 4.9 | 50000 | | 66 |
| Translation elongation fact delta | 2 spots | 4.9 | 35000 | gi\|25453472 | 93 |
| | | 5.0 | 35000 | | 70 |
| mPR | 3 spots | 4.6 | 22000 | gi\|5729875 | 95 |
| | | 4.55 | 22000 | | 110 |
| | | 4.5 | 22000 | | 107 |
| Transferrin receptor | 2 spots | 6.0 | 80000 | gi\|37747855 | 99 |
| | | 6.2 | 81000 | gi\|4557871 | 73 |
| Transgelin | 2 spots | 7.8 | 22000 | gi\|4507359 | 95 |
| | | 8.5 | 21000 | | 137 |
| Vimentin | 3 spots | 4.8 | 47000 | gi\|4507895 | 336 |
| | | 5.0 | 56000 | | 531 |
| | | 4.9 | 45000 | | 150 |

**Table 3**

| **1. Position** | **2. Sequence** | **3. Motif predicted** | **4. Consensus type** |
|---|---|---|---|
| S56* | QPAASGDSDDDEPPP (SEQ ID NO:1) | Acidophilic S/T Kinase | CK2-P |
| P62* | DSDDDEPPPLPRLKR (SEQ ID NO:2) | SH3 | Crk/Grb2/Abl/Src |
| P108 | KGRKFYGPEGPYGVF (SEQ ID NO:3) | Kinase binding | ERK1 binding |
| Y112 | FYGPEGPYGVFAGRD (SEQ ID NO:4) | Tyr-Kinase | Lck/Abl-P |
| Y138 | KEALKDEYDDLSDLT (SEQ ID NO:5) | SH2 | Shc |
| T160 | SDWESQFTFKYHHVG (SEQ ID NO:6) | Kinase binding | PDK1 binding |
| Y179* | EGEEPTVYSDEEEPK (SEQ ID NO:7) | SH2 | Fgr/SHIP |
| T180 | GEEPTVYSDEEEPKD (SEQ ID NO:8) | Acidophilic S/T Kinase | CK2-P |

## Claims

1. Use of phosphorylated and/or non-phosphorylated membrane associated progesterone receptor component 1 (mPR), in particular of at least one isoform thereof, as diagnostic marker and/or therapeutic target for diseases associated with aberrant biological phenotypes.

2. Use of at least one reagent for influencing, in particular increasing or inhibiting, the phosphorylation status (degree of phosphorylation) of membrane associated progesterone receptor component 1 (mPR), in particular of at least one isoform thereof, for diagnosis and/or therapy of diseases associated with aberrant biological phenotypes.

3. Use of at least one reagent for influencing, in particular increasing or inhibiting, the phosphorylation status (degree of phosphorylation) of membrane associated progesterone receptor component 1 (mPR), in particular of at least one isoform thereof, for the manufacture of a medicament and/or pharmaceutical composition for diagnosis and/or therapy of diseases associated with aberrant biological phenotypes.

4. Use of proteins, in particular of isoforms thereof, as diagnostic markers and/or therapeutic targets for diseases associated with aberrant biological phenotypes, **characterized in that** said proteins are involved, in particular differentially involved, in protein interaction or multi-protein complexes with either phosphorylated or non-phosphorylated membrane associated progesterone receptor component 1 (mPR).

5. Use of at least one reagent for influencing, in particular increasing or inhibiting, the abundance and/or activity of proteins, in particular of isoforms thereof, for diagnosis and/or therapy of diseases associated with aberrant biological phenotypes, **characterized in that** said proteins are involved, in particular differentially involved, in protein interaction or multi-protein complexes with either phosphorylated or non-phosphorylated membrane associated progesterone receptor component 1 (mPR).

6. Use of at least one reagent for influencing, in particular increasing or inhibiting, the abundance and/or activity of proteins, in particular of isoforms thereof, for the manufacture of a medicament and/or pharmaceutical composition for diagnosis and/or therapy of diseases associated with aberrant biological phenotypes, **characterized in that** said proteins are involved, in particular differentially involved, in protein interaction or protein complexes with either phosphorylated or non-phosphorylated membrane associated progesterone receptor component 1 (mPR).

7. Use of an assay comprising the determination of the phosphorylation status (degree of phosphorylation) of membrane associated progesterone receptor component 1 (mPR), in particular of at least one isoform thereof, in human samples for diagnosis and/or therapy of diseases associated with aberrant biological phenotypes.

8. Use according to claim 7, **characterized in that** the assay comprises the screening for reagents that are suited for diagnosis and/or therapy of diseases associated with aberrant biological phenotypes.

9. Use according to claim 7 or 8, **characterized in that** the human samples are harvested by biopsy and/or surgical extraction.

10. Use according to one of the claims 7 to 9, **characterized in that** the assay comprises the determination of proteins that are involved, in particular differentially involved, in protein interaction or multi-protein complexes with either phosphorylated or non-phosphorylated membrane associated progesterone receptor component 1 (mPR).

11. Use according to one of the claims 7 to 10, **characterized in that** the assay comprises the application of affinity reagents, in particular of antibodies.

12. Use according to one of the claims 7 to 11, **characterized in that** the assay comprises the application of mass spectrometry, in particular MALDI (Matrix Assisted Laser Desorption/lonization) and/or SELDI (Surface Enhanced Laser Desorption/lonization).

13. Use according to one of the claims 7 to 12, **characterized in that** the assay comprises the application of resonance techniques, in particular of plasma surface resonance.

14. Use according to one of the preceding claims, **characterized in that** the diseases, in particular subgroups thereof, comprise cancer, neurodegenerative diseases, infertility, inflammatory, respiratory and/or pulmonary diseases.
